Europäisches Patentamt

**European Patent Office**

Office européen des brevets

(19)

(11) Publication number: **0 105 765**
A2

(12)

# EUROPEAN PATENT APPLICATION

(21) Application number: **83306038.7**

(51) Int. Cl.³: **A 61 F 1/08**

(22) Date of filing: **05.10.83**

(30) Priority: **05.10.82 GB 8228359**

(43) Date of publication of application:
**18.04.84 Bulletin 84/16**

(84) Designated Contracting States:
**AT BE CH DE FR IT LI LU NL SE**

(71) Applicant: **UNIVERSITY COLLEGE LONDON**
**Gower Street**
**London WC1E 6BT(GB)**

(72) Inventor: **Lawrence, Reginald Bernard**
**Coppice Binton Avenue**
**Sands Nr. Farnham Surrey(GB)**

(72) Inventor: **Knox, William**
**20 Wyets Road**
**Epsom Surrey(GB)**

(74) Representative: **Boon, Graham Anthony**
**Elkington and Fife High Holborn House 52/54 High**
**Holborn**
**London, WC1V 6SH(GB)**

(54) **Lower limb prosthesis.**

(57) The invention relates to a lower limb prosthesis. The prosthesis comprises a tapered structural column (1) made from a thermoplastic material. The column has an adjustable joint (2,3) at both ends for attachment to a stump socket (4) and to a foot member (8). The adjustable joints (2,3) may be formed of opposing wedge-shaped discs (5,9) which can be locked together at the correct alignment.

EP 0 105 765 A2

- 1 -

# LOWER LIMB PROSTHESIS

The present invention relates to a lower limb prosthesis.

The two known basic prosthetic systems are an exoskeletal system and an endoskeletal nodular system.

The exoskeletal system uses a stressed skin construction of an aluminium alloy sheet shaped into a suitable structure to support the patient. An alternative exoskeletal structure is formed of a matrix of synthetic structural foam, resin, glass fibre and wood. An acceptable cosmetic shape is provided to both constructions by eye. Both constructions use a technique of static alignment which is permanently built into the prosthesis.

The endoskeletal system uses an aluminium tubular column and an assortment of add-on subunits some of which are adjustable for alignment purposes. A polyurethane foam cosmesis is formed over the column which is trimmed by eye to form a cosmetic shape.

The geat disadvantage of the exosketal system is that it is labour intensive and requires highly skilled staff. It is therefore very expensive and requires centralised fabrication. Further, since a number of fitting sessions are needed with the patient and since fabrication of the prostheses cannot occur at each limb-fitting centre, substantial delays arise.

It has also been found that while the endoskeletal system was designed to achieve simplicity of construction, a quicker delivery of prostheses, and easily adjustable, cheap and light prostheses, no substantial improvements over the exoskeletal system have been made.

- 2 -

The present invention aims to overcome or mitigate at least some of the disadvantages of the known systems.

According to the present invention there is provided a lower limb prosthesis comprising a tapered structural column formed of a thermoplastic material, the column having at its upper end an adjustable joint for connection to a socket member and at its lower end an adjustable joint for connection to a foot member.

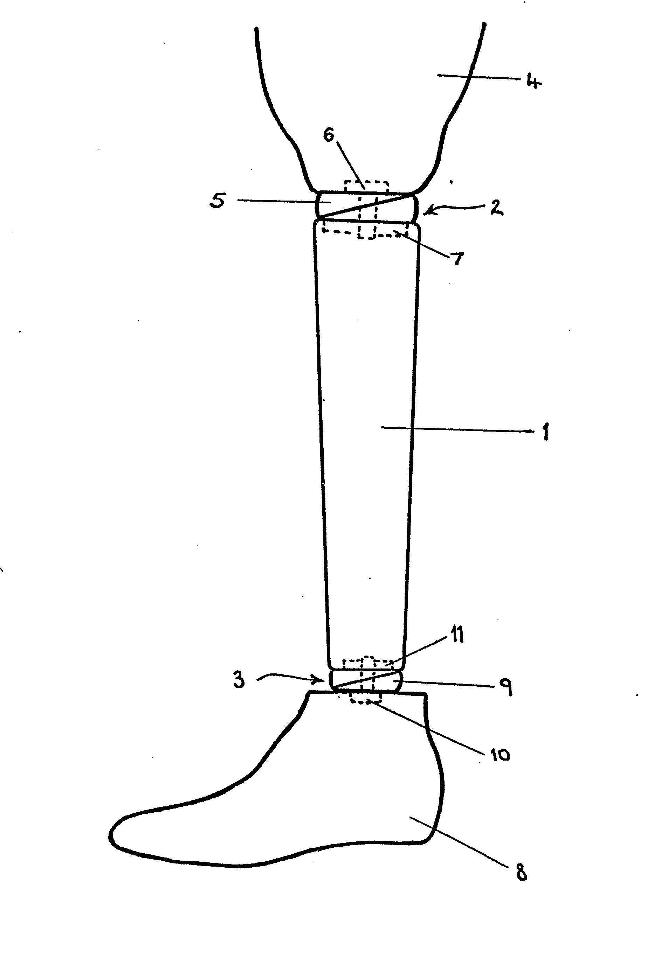

An embodiment of the present invention is described in detail below, by example only, with reference to the accompanying drawing.

The drawing shows a side elevation of a lower limb prosthesis according to the invention.

The lower limb prosthesis comprises a tapered structural column 1 having adjustable joints 2 and 3 at its upper and lower ends respectively.

The column 1 is formed of a thermoplastic material, for example nylon. The column 1 can be made by rotational casting or injection moulding. The frusto-conically shaped column 1 is preferably hollow to save weight.

The adjustable joint 2 for connection to the socket 4 comprises a pair of opposing wedge-shaped discs 5. The double wedge system allows the alignment of the column 1 with respect to the socket 4 to be adjusted. The adjustment is achieved by loosening the central bolt 6 fitting through the wedges 5 and the insert 7 in the column 1 and rotating the wedges 5 to the appropriate position. The bolt 6 is then tightened. Of course, pinning or other locking methods may be used. If pinning is used, the two wedges are aligned, then drilled and a pin is put through the eccentric drill-hole to lock the wedges.

- 3 -

The opposing surfaces of the wedges might be roughened or corrugated to prevent them from slipping relative to each other after they are locked together. Another type of adjustable joint could be used, for example a universal ball-joint connection.

The joint 3 at the lower end of the column 1 for connection to the foot member 8 is formed in a corresponding fashion to the upper joint 2, i.e. with wedges 9, bolt 10 and insert 11.

A range of angular adjustment of $\pm$ 8° is thought to be appropriate for the joints 2,3.

A simple cosmetic cover (not shown) can be pulled up over the assembled prosthesis. The cover can be a moulded flexible foam sheath, coming in a range of sizes, which can if necessary be padded out to give any desired cosmetic shape.

Thus, the invention provides a cheap, lightweight prosthesis which has the capability of rapid assembly and adjustment both during fitting and in subsequent use. In practice, a patient need only visit a limb-fitting centre once since after he has been measured up the column could be fabricated and/or fitted immediately. Sending an order to a central factory for fabrication would not be necessary with the simple prosthesis of the invention. After the correct length of column is formed, the fitting to the vacuum-formed socket and to the foot and the alignment of the prosthesis is very simple. Further, the alignment can be changed later on very simply.

If the columns are made by rotational casting in a simple semi-automatic rotational casting machine they could be made on an "as required" basis at each fitting centre. If injection moulding, which would probably be more expensive, were used it would probably be most suitable to have a stock of injection mouldings of varying lengths at each limb-fitting
centre.

- 5 -

## CLAIMS

1. A lower limb prosthesis comprising a tapered structural column (1) formed of a thermoplastic material, the column having at its upper end an adjustable joint (2) for connection to a socket member and at its lower end an adjustable joint (3) for connection to a foot member.

2. A prosthesis according to claim 1, characterised in that the tapered column (1) is hollow.

3. A prosthesis according to claim 1 or 2, characterised in that one or each adjustable joint (2,3) comprises a pair of lockable opposing wedge-shaped discs (5,9).

4. A prosthesis according to claim 3, characterised in that the discs (5,9) are lockable by a central bolt.

5. A prosthesis according to claim 3, characterised in that the discs (5,9) are lockable by an eccentric pin.

6. A prosthesis according to claim 3, characterised in that opposing surfaces of the discs (5,9) are roughened.

7. A prosthesis according to claims 1 or 2, characterised in that the or each adjustable joint comprises a universal ball-joint.